# EUROPEAN PATENT APPLICATION

(11) **EP 4 338 843 A1**
(43) Date of publication of application: **20.03.2024**
(21) Application number: 21941744.1
(22) Date of filing: 24.12.2021
(51) Int. Cl.: B01L 9/00, B01L 9/06, G01N 33/48

(54) **IN-VITRO DIAGNOSIS DEVICE AND BASKET THEREOF**

(30) Priority: 13.05.2021 CN 202110523727
(71) Applicant: Autobio Labtec Instruments Co., Ltd., Zhengzhou, Henan 450016 (CN)
(72) Inventor: SUN, Long, Zhengzhou, Henan 450016 (CN); WANG, Chao, Zhengzhou, Henan 450016 (CN); ZHAO, Peng, Zhengzhou, Henan 450016 (CN); LI, Zhenkun, Zhengzhou, Henan 450016 (CN); ZHANG, Chaohui, Zhengzhou, Henan 450016 (CN); LIU, Cong, Zhengzhou, Henan 450016 (CN)
(74) Representative: Kolster Oy Ab
(86) International application number: PCT/CN2021/141191
(87) International publication number: WO 2022/237182

(57) **Abstract**

An in-vitro diagnosis device and a basket (100). The basket (100) comprises a cover plate (102), a support assembly (107), a basket bottom plate (101) for holding at least two rows of reagent strips (400), and a sample holder (103) for holding at least two sample tubes (500); the sample holder (103) is detachably connected to an end surface of the basket bottom plate (101); the cover plate (102) and the basket bottom plate (101) are rotatably connected so as to limit displacement of the reagent strips (400) in the height direction when the cover plate (102) and the basket bottom plate (101) are attached to each other; the support assembly (107) is connected to the bottom surface of the basket bottom plate (101), and the height of the support assembly (107) is greater than or equal to the heights of the reagent strips (400). By means of the basket bottom plate (101) and the sample holder (103), the reagent strips (400) and the sample tubes (500) can be placed in batches at the same time, the cumbersome process requiring multiple placements is saved, and the placement efficiency is high; the basket (100) can accurately position the placed reagent strips (400) and the sample tubes (500), thereby avoiding re-positioning the reagent strips (400) and the sample tubes (500) when the basket is mounted on an external workbench.

## Description

The present application claims priority to Chinese Patent Application No. 202110523727.8, titled "IN-VITRO DIAGNOSIS DEVICE AND LIFTING BASKET THEREOF", filed on May 13, 2021 with the China National Intellectual Property Administration, which is incorporated herein by reference in its entirety.

### FIELD

The present application relates to the technical field of in-vitro diagnosis, and in particular to a lifting basket. In addition, an in-vitro diagnosis device having the lifting basket is also disclosed according to the present application.

### BACKGROUND

In the actual use of automatic in-vitro diagnosis device, a detector often needs to detect multiple samples in one batch. Since different types of samples require different types of detection reagents, the types of detection reagents for detection are various, and manual placement of sample tubes and reagent strips requires much labor and is inefficient.

To sum up, how to improve the placement efficiency of samples and detection reagents is an urgent problem to be solved by those skilled in the art.

### SUMMARY

The objective of the present application is to provide a lifting basket that can simultaneously hold multiple sets of sample tubes and reagent strips, which realizes batch placement of sample tubes and reagent strips and effectively improves placement efficiency of samples and detection reagents.

In addition, an in-vitro diagnosis device having the lifting basket is also provided according to the present application.

In order to achieve the above objective, the present application provides the following technical solutions.

A lifting basket includes a cover plate, a support assembly, a lifting basket bottom plate for holding at least two rows of reagent strips and a sample holder for holding at least two sample tubes, and the sample holder is detachably connected to an end face of the lifting basket bottom plate;
the cover plate is rotatably connected to the lifting basket bottom plate to restrict movement of the at least two rows of reagent strips in a height direction when the cover plate contacts the lifting basket bottom plate; and
the support assembly is connected to a bottom surface of the lifting basket bottom plate, and a height of the support assembly is greater than or equal to heights of the at least two rows of reagent strips.

In an embodiment, the lifting basket further includes a handle, and the handle is rotatably connected to the lifting basket base plate.

In an embodiment, the lifting basket bottom plate includes a handle mounting edge and multiple reagent-strip mounting holes for holding the at least two rows of reagent strips, and the handle mounting edge is arranged around the lifting basket base plate;
the handle contacts the handle mounting edge when the handle is not rotated relative to the lifting basket bottom plate.

In an embodiment, a buffer pad is provided on an upper surface of the handle mounting edge.

In an embodiment, a first magnet is provided on a top surface of the lifting basket bottom plate, a second magnet is provided on a bottom surface of the cover plate, and the first magnet is attracted to the second magnet when the cover plate contacts the lifting basket bottom plate.

In an embodiment, the lifting basket further includes a positioning assembly, the positioning assembly includes a guide seat, an extension spring, a guide shaft provided along a length direction of the lifting basket bottom plate, and a baffle for limiting the at least two rows of reagent strips, and, the guide seat is connected to the bottom surface of the lifting basket bottom plate; and
the guide shaft is slidably connected to the guide seat, one end of the guide shaft is vertically connected to the baffle, and two ends of the extension spring are respectively connected to a bottom surface of the baffle and the bottom surface of the lifting basket bottom plate.

In an embodiment, the handle includes two C-shaped handles of a same size and opposite rotation directions, and two ends of each of the two C-shaped handles are respectively rotatably connected to two ends of the lifting basket bottom plate in the length direction of the lifting basket bottom plate.

An in-vitro diagnosis device includes an external workbench and the lifting basket described above, which is mounted on the external workbench;
a buckle assembly and a pressing assembly for positioning the lifting basket are provided on the external workbench, and the buckle assembly and the pressing assembly are respectively engaged with two ends of the lifting basket in the length direction of the lifting basket.

In an embodiment, the buckle assembly includes a buckle seat connected to the external workbench, a buckle rotatably connected to the buckle seat and a first compression spring, and, a buckle groove is formed in the middle of the buckle seat; and
one end of the first compression spring is connected to the buckle, and the other end of the first compression spring is connected to a bottom surface of the buckle groove.

In an embodiment, the pressing assembly includes a pressing fixing seat, a spring press block, a second compression spring, and a press-block cover plate, and, the pressing fixing seat is connected to the external workbench;
a spring-press-block groove for mounting the spring press block and the second compression spring is formed on a side surface of the pressing fixing seat, and the press-block cover plate is connected to an upper end face of the spring-press-block groove; and
one end of the second compression spring is connected to the spring press block, and the other end of the second compression spring is connected to a bottom surface of the spring-press-block groove.

When sample tubes and reagent strips are required to be placed in the lifting basket according to the present application, the sample tubes are first placed into the sample holder one by one, and then the cover plate is turned to separate the cover plate from the lifting basket bottom plate, and the reagent strips are placed into the lifting basket bottom plate. After the reagent strips are placed, the cover plate is turned to attach the cover plate to the lifting basket bottom plate, so as to prevent the reagent strips from moving in the height direction. The lifting basket is placed on the external workbench of the in-vitro diagnosis device and fixed.

After the detection is completed, the lifting basket is removed from the external workbench.

Therefore, the lifting basket according to the present application realizes the simultaneous placement of the reagent strips and the sample tubes in batches through the lifting basket bottom plate and the sample holder, which saves the cumbersome process of multiple placements and has high placement efficiency. The lifting basket can position the reagent strips and the sample tubes placed therein, and the positioning is accurate, thereby avoiding repositioning the reagent strips and the sample tubes after the lifting basket is placed on the external workbench.

In addition, an in-vitro diagnosis device having the lifting basket described above is also provided according to the present application.

### BRIEF DESCRIPTION OF THE DRAWINGS

In order to more clearly illustrate the technical solutions in the embodiments of the present application or in the conventional technology, the following will briefly introduce drawings required in the description of the embodiments or the conventional technology. Apparently, the drawings in the following description are only the embodiments of the present application. For those skilled in the art, other drawings can also be obtained based on the provided drawings without creative efforts.
Figure 1 is a schematic exploded view of a lifting basket according to an embodiment of the present application;
Figure 2 is a schematic diagram of a mechanism after a lifting basket and an external workbench are assembled according to an embodiment of the present application;
Figure 3 is a schematic structural diagram of a buckle assembly of an in-vitro diagnosis device according to an embodiment of the present application;
Figure 4 is a schematic structural diagram of a pressing assembly of an in-vitro diagnosis device according to an embodiment of the present application;
Figure 5 is a schematic structural diagram of a pressing fixing seat and a second compression spring in the pressing assembly in Figure 4; and
Figure 6 is a schematic structural diagram of a spring press block in the pressing assembly in Figure 4.

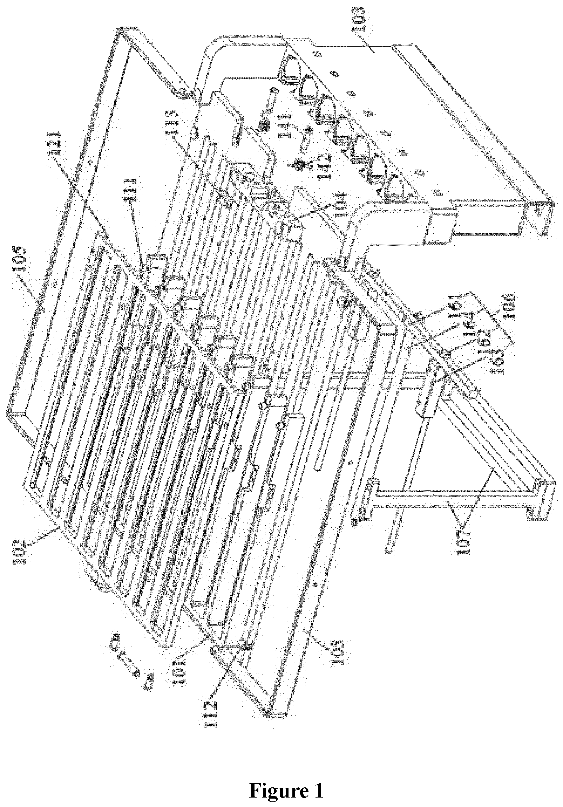

Reference signs in Figures 1 to 6 are listed as follows:
100 lifting basket, 101 lifting basket bottom plate, 111 first magnet, 112 buffer pad, 113 insert, 102 cover plate, 121 second magnet, 103 sample seat, 104 handle fixing seat, 141 handle rotating shaft, 142 torsion spring, 105 handle, 106 positioning assembly, 161 baffle, 162 guide shaft, 163 guide seat, 164 extension spring, 107 support assembly, 200 buckle assembly, 201 buckle seat, 202 buckle, 203 first compression spring, 300 pressing assembly, 301 pressing fixing seat, 302 spring press block, 303 second compression spring, 304 press-block cover plate, 400 reagent strip, and 500 sample tube.

### DETAILED DESCRIPTION OF EMBODIMENTS

The technical solutions in the embodiments of the present application are clearly and completely described below in conjunction with the drawings. Of course, the described embodiments are only a part of the embodiments of the present application, not all embodiments. All other embodiments obtained by those skilled in the art based on the embodiments in the present application without creative efforts shall fall within the protection scope of the present application.

The core of the present application is to provide a lifting basket that can place multiple sets of sample tubes and reagent strips at the same time, which realizes batch placement of sample tubes and reagent strips, and effectively improves the placement efficiency of samples and detection reagents.

An in-vitro diagnosis device having the lifting basket described above is also provided according to the present application.

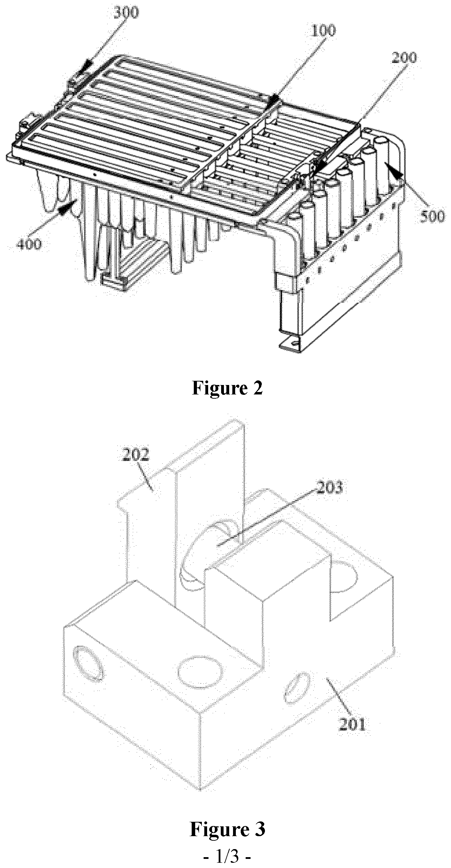

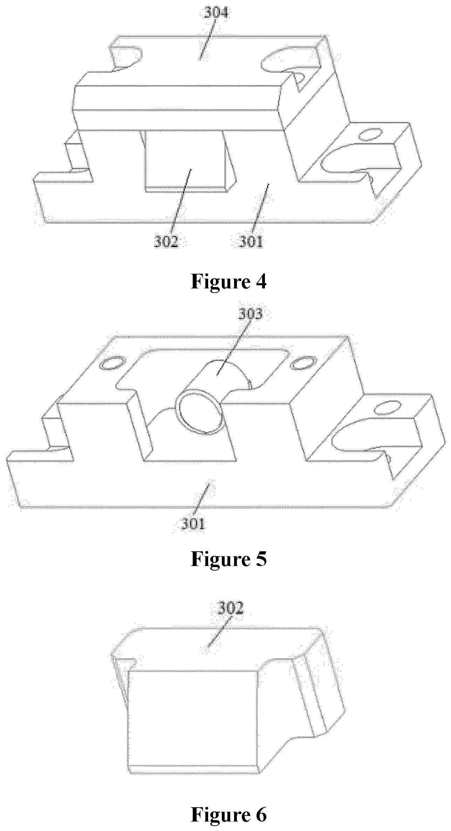

Referring to Figures 1 to 6, Figure 1 is schematic exploded view of a lifting basket according to an embodiment of the present application; Figure 2 is a schematic diagram of a mechanism after a lifting basket and an external workbench are assembled according to an embodiment of the present application; Figure 3 is a schematic structural diagram of a buckle assembly of an in-vitro diagnosis device according to an embodiment of the present application; Figure 4 is a schematic structural diagram of a pressing assembly of an in-vitro diagnosis device according to an embodiment of the present application; Figure 5 is a schematic structural diagram of a pressing fixing seat and a second compression spring in the pressing assembly in Figure 4; and Figure 6 is a schematic structural diagram of a spring press block in the pressing assembly in Figure 4.

The lifting basket 100 according to an embodiment includes a cover plate 102, a support assembly 107, a lifting basket bottom plate 101 for holding at least two rows of reagent strips 400, and a sample holder 103 for holding at least two sample tubes 500. The sample holder 103 is detachably connected to an end face of the lifting basket bottom plate 101. The cover plate 102 is rotatably connected to the lifting basket bottom plate 101 to restrict movement of each reagent strip 400 in a height direction when the cover plate 102 contacts the lifting basket bottom plate 101. The support assembly 107 is connected to a bottom surface of the lifting basket bottom plate 101, and a height of the support assembly 107 is greater than or equal to heights of the at least two rows of reagent strips 400.

Referring to Figure 1, multiple reagent-strip mounting holes arranged in parallel are formed in the middle of the lifting basket bottom plate 101. The multiple reagent-strip mounting holes are arranged along a length direction of the lifting basket bottom plate 101, and sizes of the multiple reagent-strip mounting holes can be determined based on sizes of the reagent strips 400. Multiple sample-tube mounting holes are formed in the sample holder 103, and sizes of the multiple sample-tube mounting holes can be determined based on outer diameters of the sample tubes 500. The number of the multiple reagent-strip mounting holes and the number of the multiple sample-tube mounting holes determine a maximum number of reagent strips 400 and a maximum number of sample tubes 500 that can be placed in the lifting basket 100 at the same time, respectively. The specific numbers of the multiple reagent-strip mounting holes and the multiple sample-tube mounting holes can be determined based on actual detection requirements.

The cover plate 102 is rotatably connected to the lifting basket bottom plate 101, so as to prevent the reagent strips 400 from moving in the height direction of the lifting basket 100, and to avoid any deviation in the positioning of the reagent strips 400 during an automatic detection process of an in-vitro diagnosis device caused by lift or disengagement of the reagent strips 400.

Referring to Figure 1, the cover plate 102 is rotatably connected to one end of the lifting basket base plate 101 in the length direction of the lifting basket base plate 101. The cover plate 102 has multiple reagent-strip positioning holes in one-to-one correspondence with the multiple reagent-strip mounting holes.

In an embodiment, one of a bottom surface of the cover plate 102 and a bottom surface of the lifting basket bottom plate 101 has a positioning groove, and the other of the bottom surface of the cover plate 102 and the bottom surface of the lifting basket bottom plate 101 has a positioning block engaged with the positioning groove. When the cover plate 102 contacts the lifting basket bottom plate 101, the positioning block engages with the positioning groove, which can effectively limit the movement of the reagent strips 400 in the height direction.

In order to simplify the structure and facilitate manufacture, in an embodiment, a first magnet 111 is provided on a top surface of the lifting basket bottom plate 101, and a second magnet 121 is provided on a bottom plate of the cover plate 102. When the cover plate 102 contacts the lifting basket bottom plate 101, the first magnet 111 is attracted to the second magnet 121. Therefore, when the reagent strip 400 is lifted or disengaged, it is necessary to overcome a suction force between the first magnet 111 and the second magnet 121, so as to realize effective limit and accurate positioning of the reagent strip 400 in the height direction of the lifting basket 100.

Lower end faces of the multiple reagent strips 400 are uneven, in order to place the lifting basket 100 on a workbench, a support assembly 107 is provided on the bottom surface of the lifting basket bottom plate 101. Specifically, the support assembly 107 may be of an H-shaped structure as shown in Figure 1, or may be support columns provided at four corners of the lifting basket bottom plate 101, or may be other conventional support structures.

A height of support assembly 107 is greater than or equal to heights of the reagent strips 400, so as to prevent the lower end faces of the reagent strips 400 from directly contacting a surface of the workbench.

When the reagent strips 400 and the sample tubes 500 are required to be placed, the sample tubes 500 are first placed into the sample holder 103 one by one, then the cover plate 102 is turned to separate the cover plate 102 from the lifting basket bottom plate 101, and the reagent strips 400 are placed into the lifting basket bottom plate 101. After the reagent strips 400 are placed, the cover plate 102 is turned to allow the cover plate 102 to contact the lifting basket base plate 101, so as to prevent the reagent strips 400 from moving in the height direction. The lifting basket 100 is placed on the external workbench of the in-vitro diagnosis device and is fixed.

After detection is completed, the lifting basket 100 is removed from the external workbench.

In the embodiment, the simultaneous placement of the reagent strips 400 and the sample tubes 500 in batches is realized through the lifting basket bottom plate 101 and the sample holder 103, which saves the cumbersome process of multiple placements and has high placement efficiency. The lifting basket 100 can position the reagent strips 400 and the sample tubes 500 placed therein, and the positioning is accurate, thereby avoiding repositioning the reagent strips 400 and the sample tubes 500 after the lifting basket 100 is placed on the external workbench.

On the basis of the above embodiments, in order to facilitate the transfer of the lifting basket 100 and improve the transfer efficiency, a handle 105 may also be provided, and the handle 105 is rotatably connected to the lifting basket bottom plate 101.

The handle 105 may be rotatably connected to the lifting basket bottom plate 101 through a pin shaft or the like. Referring to Figure 1, a handle fixing seat 104 is provided on the lifting basket bottom plate 101, a handle mounting hole is formed in the handle fixing seat 104, and the handle 105 is mounted in the handle mounting hole through a handle rotating shaft 141.

In an embodiment, referring to Figure 1, the handle 105 may include two C-shaped handles of a same size and opposite rotation directions, and two ends of each C-shaped handle are respectively rotatably connected to two ends of the lifting basket bottom plate 101 in the length direction of the lifting basket bottom plate 101.

After the reagent strips 400 and the sample tubes 500 are placed into the lifting basket bottom plate 101, the handle 105 is turned, and a detector transfers the lifting basket 100 to the external workbench of the in-vitro diagnosis device through the handle 105. After the position of the lifting basket 100 relative to the external workbench is fixed, the handle 105 is turned to be reset, so as to prevent the handle 105 from colliding the in-vitro diagnosis device, which otherwise affects a subsequent detection process. After the detection is completed, the handle 105 is turned to remove the lifting basket 100 from the external workbench.

In the embodiment, the arrangement of the handle 105 allows the detector to operate the lifting basket 100 with one hand, thereby facilitating the placement and the removal of the lifting basket 100, and improving transfer efficiency.

When the in-vitro diagnosis device detects the reagent strips 400 and the sample tubes 500 in the lifting basket 100, in order to prevent the handle 105 from colliding the in-vitro diagnosis device, the handle 105 is required to be lowered, so that an included angle between the handle 105 and the lifting basket bottom plate 101 is zero.

In order to prevent the detector from forgetting to lower the handle 105, in an embodiment, a torsion spring 142 is provided on the handle rotating shaft 141, so that the handle 105 can be automatically reset under the action of the torsion spring 142, thereby effectively preventing collision accidents during the operation of the device.

In order to save mounting space, in an embodiment, the lifting basket bottom plate 101 has a handle mounting edge and reagent-strip mounting holes for holding the reagent strips 400. The handle mounting edge is arranged around the lifting basket bottom plate 101. When the handle 105 is not rotated relative to the lifting basket bottom plate 101, the handle 105 contacts the handle mounting edge.

In order to reduce the impact when the handle 105 falls, in an embodiment, a buffer pad 112 is provided on an upper surface of the handle mounting edge. Alternatively, the buffer pad 112 may also be replaced with a part having similar functions such as a spring plunger.

After the lifting basket 100 is mounted in the in-vitro diagnosis device, a positioning member of the in-vitro diagnosis device abuts against the top surface of the lifting basket bottom plate 101. In an embodiment, an insert 113 may be provided at a position of the lifting basket bottom plate 101 where the lifting basket bottom plate 101 cooperates with the in-vitro diagnosis device. The strength and the wear resistance of the insert 113 are much higher than those of the lifting basket bottom plate 101, so that the insert 113 can withstand the impact and the wear when the lifting basket bottom plate 101 cooperates with the in-vitro diagnosis device, thereby greatly extending the service life of the lifting basket bottom plate 101.

The material, the shape, and the size of the insert 113 can be determined based on the actual testing requirements with reference to the prior art, which are not described here.

In general, placement positions of the reagent strips 400 in the lifting basket 100 are required to be fixed and consistent, and the placement positions of the reagent strips 400 may be inconsistent in the case of manual operation errors.

On the basis of the above embodiments, the lifting basket further includes a positioning assembly 106. The positioning assembly 106 includes a guide seat 163, an extension spring 164, a guide shaft 162 provided along the length direction of the lifting basket bottom plate 101, and a baffle 161 for limiting the reagent strips 400. The guide seat 163 is connected to the bottom surface of the lifting basket bottom plate 101. The guide shaft 162 is slidably connected to the guide seat 163, one end of the guide shaft 162 is vertically connected to the baffle 161, and two ends of the extension spring 164 are respectively connected to the baffle 161 and the bottom surface of the lifting basket bottom plate 101.

The baffle 161 is configured to push the reagent strips 400 to move in the length direction of the lifting basket bottom plate 101. The guide shaft 162 is slidably connected to the guide seat 163 to guide the baffle 161 and ensure that the moving direction of the baffle 161 is parallel to the length direction of the lifting basket bottom plate 101. The extension spring 164 is configured to help the baffle 161 to be reset without external force.

Referring to Figure 1, an end, which is close to a rotating shaft of the lifting basket bottom plate 101 and the cover plate 102, of the lifting basket bottom plate 101 is a front end of the lifting basket bottom plate 101. When the reagent strips 400 are being placed, the extension spring 164 is manually pulled to drive the baffle 161 to move towards the front end of the lifting basket bottom plate 101. The baffle 161 is located on the right side of the reagent strips 400, so the baffle 161 pushes the reagent strips 400 to move towards the front end of the lifting basket bottom plate 101 until the reagent strips 400 contact left wall surfaces of the reagent-strip mounting holes. At this time, the placement positions of the reagent strips 400 are consistent.

In the embodiment, fast and accurate positioning of the reagent strips 400 is realized through the positioning assembly 106, which ensures the consistency of the placement positions of the reagent strips 400.

In addition, the guide seat 163 may also be replaced with a linear bearing.

In addition to the lifting basket 100 described above, an in-vitro diagnosis device including the lifting basket 100 disclosed by the above embodiments is also provided according to the present application. The in-vitro diagnosis device includes an external workbench and the lifting basket 100, which is disclosed in the above embodiments, mounted on the external workbench. A buckle assembly 200 and a pressing assembly 300 for positioning the lifting basket 100 are provided on the external workbench. The buckle assembly 200 and the pressing assembly 300 are respectively engaged with two ends of the lifting basket 100 in the length direction of the lifting basket 100.

The pressing assembly 300 is mainly configured to limit the movement of the lifting basket 100 in the height direction, and the buckle assembly 200 is configured to fix the position of the lifting basket 100 to prevent the buckle assembly 200 from sliding relative to a surface of the external workbench.

In an embodiment, the buckle assembly 200 may include a buckle seat 201 connected to the external workbench, a buckle 202 rotatably connected to the buckle seat 201 and a first compression spring 203. A buckle groove is formed in the middle of the buckle seat 201. One end of the first compression spring 203 is connected to the buckle 202, and the other end of the first compression spring 203 is connected to a bottom surface of the buckle groove. Therefore, the buckle 202 can contract inward along the buckle groove and rotate within a certain range when the buckle 202 is pressed, and the buckle 202 is reset under the action of the first compression spring 203 when there is no external force.

In an embodiment, the pressing assembly 300 includes a pressing fixing seat 301, a spring press block 302, a second compression spring 303, and a press-block cover plate 304. The pressing fixing seat 301 is connected to the external workbench. A spring-press-block groove for mounting the spring press block 302 and the second compression spring 303 is formed on a side surface of the pressing fixing seat 301, and the press-block cover plate 304 is connected to an upper end face of the spring-press-block groove. One end of the second compression spring 303 is connected to the spring press block 302, and the other end of the second compression spring 303 is connected to a bottom surface of the spring-press-block groove. Therefore, the spring press block 302 can move inward along the spring-press-block groove when a force is applied to the spring press block 302, and the spring press block 302 can be reset under the action of the second compression spring 303 when no force is applied to the spring press block 302.

Referring to Figures 1 and 4, the spring press block 302 is configured to press the lifting basket bottom plate 101 against the surface of the external workbench, so a distance between a lower surface of the spring press block 302 and a lower surface of the pressing fixing seat 301 is greater than or equal to a thickness of the lifting basket bottom plate 101.

In an embodiment, a positioning hole is formed in the lifting basket base plate 101, and a positioning pin that fits with the positioning hole is provided on the external workbench, so as to facilitate the positioning and the mounting of the lifting basket 100.

In an embodiment, referring to Figure 2, two pressing assemblies 300 are provided on a left side of the external workbench, and one buckle assembly 200 is provided on the right side of the external workbench. The two pressing assemblies 300 are evenly distributed along a width direction of the lifting basket 100, and the buckle 202 is arranged at a position of 1/2 width of the lifting basket 100.

When the lifting basket is being mounted, first, the buckle 202 is pressed and rotated to place the lifting basket base plate 101 under the spring press block 302 of the pressing assembly 300. Then, the buckle 202 is released, the buckle 202 is extended outward under the action of the first compression spring 203, the lifting basket bottom plate 101 is pushed towards an end close to the pressing assembly 300 until the lifting basket bottom plate 101 abuts against an inner side surface of the pressing fixing seat 301. At this time, the lifting basket 100 is fixed to the external workbench by the buckle assembly 200 and the pressing assembly 300.

When the lifting basket 100 is required to be removed, the handle 105 is turned to drive the lifting basket 100 to move along the width direction of the lifting basket 100 until the lifting basket 100 is disengaged from below the spring press block 302.

It should be noted that first and second in the first magnet 111, the second magnet 121, the first compression spring 203 and the second compressed spring 303 mentioned in the present application, are only used to distinguish different positions and do not contain restrictions of order.

The embodiments of the present application are described in a progressive manner. Each embodiment focuses on the difference from other embodiments, and same or similar parts of the embodiments can refer to each other.

The above provides a detailed introduction to the in-vitro diagnosis device and the lifting basket thereof according to the present application. Specific embodiments are used herein to illustrate the principles and the implementations of the present application, and the descriptions of the above embodiments are only used to help understand methods and core ideas of the present application. It should be pointed out that for those skilled in the art, some improvements and modifications may be made to the present application without departing from the principles of the present application, and these improvements and modifications shall fall within the protection scope of the claims of the present application.

## Claims

1. A lifting basket, comprising a cover plate (102), a support assembly (107), a lifting basket bottom plate (101) for holding at least two rows of reagent strips (400), and a sample holder (103) for holding at least two sample tubes (500), wherein
the sample holder (103) is detachably connected to an end face of the lifting basket bottom plate (101);
the cover plate (102) is rotatably connected to the lifting basket bottom plate (101) to restrict movement of each of the at least two rows of reagent strips (400) in a height direction when the cover plate (102) contacts the lifting basket bottom plate (101); and
the support assembly (107) is connected to a bottom surface of the lifting basket bottom plate (101), and a height of the support assembly (107) is greater than or equal to heights of the at least two rows of reagent strips (400).

2. The lifting basket according to claim 1, further comprising a handle (105), wherein the handle (105) is rotatably connected to the lifting basket base plate (101).

3. The lifting basket according to claim 2, wherein
the lifting basket base plate (101) has a handle mounting edge and a plurality of reagent-strip mounting holes for holding the at least two rows of reagent strips (400), and the handle mounting edge is arranged around the lifting basket base plate (101);
the handle (105) contacts the handle mounting edge when the handle (105) is not rotated relative to the lifting basket bottom plate (101).

4. The lifting basket according to claim 3, wherein a buffer pad (112) is provided on an upper surface of the handle mounting edge.

5. The lifting basket according to claim 4, wherein a first magnet (111) is provided on a top surface of the lifting basket bottom plate (101), a second magnet (121) is provided on a bottom surface of the cover plate (102), and the first magnet (111) is attracted to the second magnet (121) when the cover plate (102) contacts the lifting basket bottom plate (101).

6. The lifting basket according to any one of claims 2 to 5, further comprising a positioning assembly (106), wherein
the positioning assembly (106) comprises a guide seat (163), an extension spring (164), a guide shaft (162) provided along a length direction of the lifting basket bottom plate (101), and a baffle (161) for limiting the at least two rows of reagent strips (400), the guide seat (163) is connected to the bottom surface of the lifting basket bottom plate (101); and
the guide shaft (162) is slidably connected to the guide seat (163), one end of the guide shaft (162) is vertically connected to the baffle (161), and two ends of the extension spring (164) are respectively connected to the baffle (161) and the bottom surface of the lifting basket bottom plate (101).

7. The lifting basket according to claim 6, wherein the handle (105) comprises two C-shaped handles of a same size and opposite rotation directions, and two ends of each of the two C-shaped handles are respectively rotatably connected to two ends of the lifting basket bottom plate (101) in the length direction of the lifting basket bottom plate (101).

8. An in-vitro diagnosis device, comprising an external workbench and the lifting basket (100) according to any one of claims 1 to 7, which is mounted on the external workbench, wherein
a buckle assembly (200) and a pressing assembly (300), which are configured to position the lifting basket (100), are provided on the external workbench, and the buckle assembly (200) and the pressing assembly (300) are respectively engaged with two ends of the lifting basket (100) in the length direction of the lifting basket (100).

9. The in-vitro diagnosis device according to claim 8, wherein the buckle assembly (200) comprises a buckle seat (201) connected to the external workbench, a buckle (202) rotatably connected to the buckle seat (201) and a first compression spring (203), wherein a buckle groove is formed in the middle of the buckle seat (201); and
one end of the first compression spring (203) is connected to the buckle (202), and the other end of the first compression spring (203) is connected to a bottom surface of the buckle groove.

10. The in-vitro diagnosis device according to claim 9, wherein
the pressing assembly (300) comprises a pressing fixing seat (301), a spring press block (302), a second compression spring (303), and a press-block cover plate (304), wherein the pressing fixing seat (301) is connected to the external workbench;
a spring-press-block groove for mounting the spring press block (302) and the second compression spring (303) is formed on a side surface of the pressing fixing seat (301), and the press-block cover plate (304) is connected to an upper end face of the spring-press-block groove; and
one end of the second compression spring (303) is connected to the spring press block (302), and the other end of the second compression spring (303) is connected to a bottom surface of the spring-press-block groove.
